(19) Europäisches Patentamt
European Patent Office
Office européen des brevets

(11) **EP 2 067 032 B1**

(12) **EUROPEAN PATENT SPECIFICATION**

(45) Date of publication and mention of the grant of the patent:
**21.10.2020  Bulletin 2020/43**

(21) Application number: **07859587.3**

(22) Date of filing: **17.07.2007**

(51) Int Cl.:
*G01N 33/20* (2019.01)     *B01L 3/04* (2006.01)

(86) International application number:
**PCT/IN2007/000295**

(87) International publication number:
**WO 2008/038297 (03.04.2008 Gazette 2008/14)**

(54) **AN APPARATUS AND METHOD FOR DETERMINING THE PERCENTAGE OF CARBON EQUIVALENT, CARBON AND SILICON IN LIQUID FERROUS METAL**

VORRICHTUNG UND VERFAHREN ZUR BESTIMMUNG DES PROZENTUALEN ANTEILS EINES KOHLENSTOFFÄQUIVALENTS SOWIE VON KOHLENSTOFF UND SILICIUM IN FLÜSSIGEM EISENMETALL

PROCÉDÉ ET APPAREIL DE DÉTERMINATION DU POURCENTAGE DE L'ÉQUIVALENT CARBONE, DU CARBONE ET DU SILICIUM DANS UN MÉTAL FERREUX LIQUIDE

(84) Designated Contracting States:
**AT BE BG CH CY CZ DE DK EE ES FI FR GB GR HU IE IS IT LI LT LU LV MC MT NL PL PT RO SE SI SK TR**

(30) Priority:  **29.09.2006  IN MU15842006**

(43) Date of publication of application:
**10.06.2009  Bulletin 2009/24**

(73) Proprietors:
• **Kakatkar, Anant Kashinath**
  **Pune 411 037 Maharashtra (IN)**
• **Kelkar, Satish Shashikant**
  **Pune 411 037 Maharashtra (IN)**

(72) Inventors:
• **Kakatkar, Anant Kashinath**
  **Pune 411 037 Maharashtra (IN)**
• **Kelkar, Satish Shashikant**
  **Pune 411 037 Maharashtra (IN)**

(74) Representative: **Lee, Nicholas John et al**
**Kilburn & Strode LLP**
**Lacon London**
**84 Theobalds Road**
**London WC1X 8NL (GB)**

(56) References cited:
**US-A- 4 515 485     US-A- 5 503 475
US-A- 5 720 553**

## Description

### Technical Field:

The present invention relates to an apparatus and method for determining the percentage of Carbon Equivalent, Carbon and Silicon in liquid ferrous metal. More particularly, the present invention relates to the detection of the composition of liquid ferrous metal in a much quicker time using refractory cups.

### Background and Prior Art:

Existing thermal analysis for detection of percentage of carbon equivalent, carbon and silicon requires about 180 seconds and consumes about 200- 325 grams of metal. Thermal analysis involves measurement and analysis of cooling pattern of the liquid metal, under controlled conditions. When liquid metal is poured in a cup, the maximum temperature as recorded by the cup is stored and further slow cooling is scanned. During cooling, when first nucleus solidifies, it gives away its kinetic energy, a thermal arrest is seen, which is metallurgically called as liquidus temperature (TL). This temperature being inversely proportional to the percentage of carbon equivalent (CE), helps in determining the %CE value. On further cooling, with the help of tellurium coating at the base of the cup, the sample is chilled and converted into white iron, instead of grey. This gives the solidification temperature (TS). The detection of TL and TS helps in detecting the percentage of Carbon and silicon.

US5503475 relates to a method for determining the carbon equivalent, carbon content and silicon content of molten cast iron.

US5720553 relates to an apparatus and process for rapid direct dip analysis of molten iron.

US4,358,948 discloses a molten metal crucible comprising a horizontal thermocouple (32) provided at its base.

The existing cups used are round or square in shape such that 225- 325 grams of liquid metal can be accommodated for testing. Tellurium is pasted at the bottom of the cup or is coated all over the inner surface of the cup. The thermocouple wire used for measuring the temperature is a thick K type (CR-AL) wire of 22 SWG.

US patent 3404570 discloses the method and apparatus for determining the concentration of a silicon in a sample of an electrically conductive material by measuring in a poured sample of the material cooling in a manner such that a temperature gradient exists across the sample, the temperature and a thermocouple force produced with the sample at each of two points in the sample spaced apart in the direction of the temperature gradient. The magnitude of the difference between the electromotive forces at a predetermined temperature difference between the points is representative of the concentration of the constituent.
However this method is complex and time consuming.

The present invention is fast and simple and takes only 50 to 180 seconds. The concept of finding composition in present invention is by thermal analysis as against conductivity measurement in prior art.

US Patent No.3546921 discloses a method of producing an initial thermal arrest in the cooling curve of a molten sample of hypereutectic cast iron by addition of carbide stabilizers as Bi, B, Ce etc. are invented to get consistent discernible thermal arrest.

However this method applies only for determination of only Carbon Equivalent by determination of Liquidus temperature only. This invention does not determine % Carbon and % Silicon in the metal.

The present invention determines one more thermal arrest called solidus temperature. Moreover two additional elements viz. Carbon and silicon are determined as against only Carbon equivalent in prior art.

US Patent No.4059996 sets forth an improvement over the other by disclosing a blob of material in contact with the bottom wall of a cavity. The blob of material includes a carbide formation promoting material and preferably mixed with a material for evolving hydrogen. The refractory material aids in preventing the carbide formation promoting material from being burned up quickly and mixing too quickly with the molten metal. The hydrogen thus evolved is used to generate turbulence in metal that help carbide forming material to reach to every corner of cup and thus achieve formation of carbides all over the cup.

The problem with this method is due to turbulence, the temperature drop is observed while filling the cup and cup filling is a skilled job. A stop and repeat pouring practice has to be followed to stop the boiling metal from coming out of the cup. All this is aimed at having uniform spreading of chilling agents throughout the cup.

In the present invention, turbulence is not created. The carbide forming material gets mixed without external force because of smaller volume of cup. This avoids generation of harmful hydrogen at such a high temperature of $1400^0$ C and splashing of metal from cup.

US Patent No. 4515485 also describe the improvement in U.S Patent No.4059996 for mixing of chilling agents through out the cup by using evolved hydrogen in a better controlled fashion.
However it does not completely solve the problems associated with boiling and spilling of metal due to generation of hydrogen.

The present invention removes from root, the cause of creating turbulence by reducing the volume of cup that eliminates the requirement of generation of turbulence in the liquid metal.

US Patent No. 4274284 describe a method to improve response time of Cromel Alumel thermocouple that is used to measure temperature of the cup. High response time is very essential for accurate measurement of thermal arrests as described therein. The thermocouple is under constant thermal stress till analysis is complete. This necessitates the use of thicker gauge causing response time and higher cost of wire and hence measurement. The gauge of the wire is more to withstand thermal stress as time requires for measurement is large.

However, the thermocouple remains exposed to liquid metal and thus the metal contaminates the thermocouple hampering accuracy.

In the present invention, volume of sample is reduced to 50 to 180 grams as against 200- 325 grams as required by the prior art. Due to lower sampling time, the time for which the thermocouple has to undergo thermal stress reduces. A thinner thermocouple can be used due to lower exposure time. Secondly, a thinner wire has lesser lag and hence better response time. Hence objective of the prior art to reduce temperature lag is achieved automatically by reducing diameter of wire. The present invention allows using thinner thermocouples thereby reducing cost of sampling. The quartz tube (3) used eliminates contamination of carbonaceous material, which is another objective of prior art.

US Patent No.6739750 provides a sampling vessel for thermal analysis of molten metal by reducing the time required with the help of probe type sampling vessel. The volume of the vessel is decided by the limitation in measurement accuracy of cooling rate. The cooling rate is required to be closer to (0 to - 0.20 as mentioned in the figure 3 B). In the said process the conventional diameter of around 30mm was reduced to around 20 mm and conventional depth of 50 mm was reduced to 36 mm or more.

The use of this technique involves the use of costlier probe type sample having a limitation of minimum depth of 36 mm of cavity.

With the present invention, by hardware and algorithm, cooling rate upto $3^0$ C is measured instead of $0.20^0$ C, which eliminate the limitation of depth of 36 mm or more in prior art.

US Patent No.5720553 describe the use of metallic inserts, instead of chilling agents, to act as a heat sink thereby promoting white solidification.

However, the cost of measurement is high and technique involves immersion type of sampling which is not preferred for measurement everywhere.

The present invention uses chilling agents and low volume of sample metal for promoting white solidification.

**Drawbacks of Prior Art:**

1. The metal solidifies in the patches of grey and white iron which hampers accuracy of the testing to a great extent.
2. The pouring temperature of the metal is very high. It burns off some amount of tellurium thereby affecting quality of test.
3. The thermocouple is under constant thermal stress till analysis is complete.
4. The metal is held in the furnace for longer time which results in loss of electricity/power and deteriorates the quality of metal.
5. The thermal analysis requires more time.
6. The quantity of metal required for analysis is more.
7. The quantity of chilling agent required is more.

**Summary of the Present Invention:**

The main object of the present invention is to provide;

A) A method using refractory cup made from resin coated sand having capacity of 50 to 180gm instead of prior art cup which needs quantity of 200-325 gm.
B) Hardware for determination of percentage of carbon, silicon and carbon equivalent.

Another object of the present invention is to increase the cooling rate by reducing the size of the resin coated cups. Lesser the volume, higher is the surface area to weight ratio and hence higher cooling rate is achieved.

Still further object of the present invention is to achieve balance in the pouring temperature such that temperature and time required is available for mixing of chilling agents and at the same time maximum cooling rate is achieved.

The purpose of the present invention is to reduce time needed for chilling material to mix at every corner of the cup in short time by reducing the distance of edges from centre of the cup by reducing the dimensions of the cup.

The aim of the present invention is to save time i.e. 50 to 80 seconds as against prior art, which needs 180 seconds and to save metal taken in the cavity for thermal analysis.

**Advantages of the Present Invention:**

1. The metal solidifies into white iron as cooling rate is increased by reducing size of the cup and thereby reducing volume of the liquid metal which helps in accuracy of the testing.
2. The stress on the thermocouple last for a lesser time as time required for analysis is reduced due to faster cooling rate.
3. The time required for chilling material (tellurium) to mix at every corner of the cup is reduced as dimensions of the cup are changed.
4. The metal is held in furnace for shorter duration thereby saving electricity/power and helps in maintaining the quality of metal.
5. The quantity of metal required for analysis is less and thereby decrease in wastage of metal.
6. The quantity of chilling agents to convert gray iron to white iron is reduced.
7. The present invention thus provides convenient and rapid method for thermal analysis of a liquid ferrous metal.

**Description of the Present Invention:**

According to the present invention for thermal analysis of a liquid ferrous metal, there is provided an apparatus and method for determining the concentration of a constituent in a liquid ferrous metal. More particularly present invention relates to a method and apparatus for determination of percentage of Carbon, Silicon and Carbon equivalent using electronic equipment.

**Apparatus:**

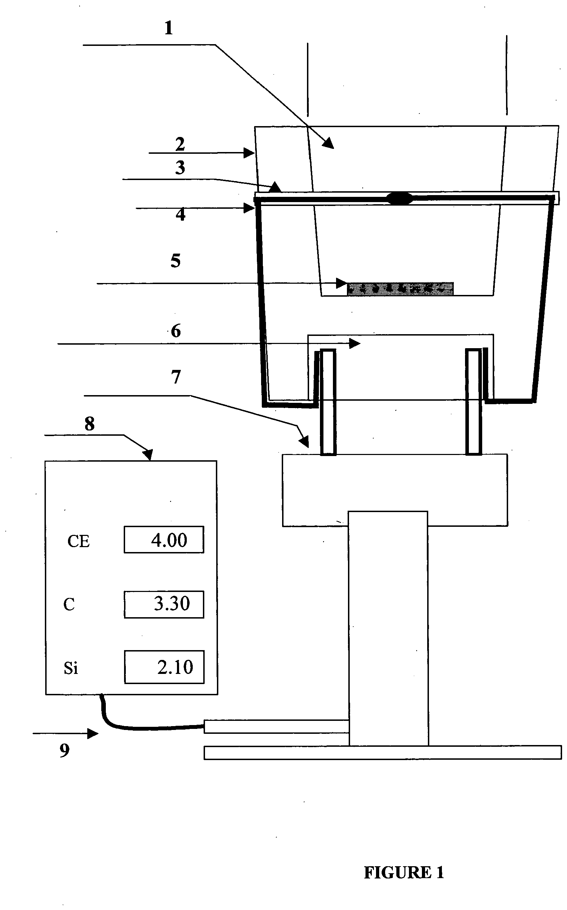

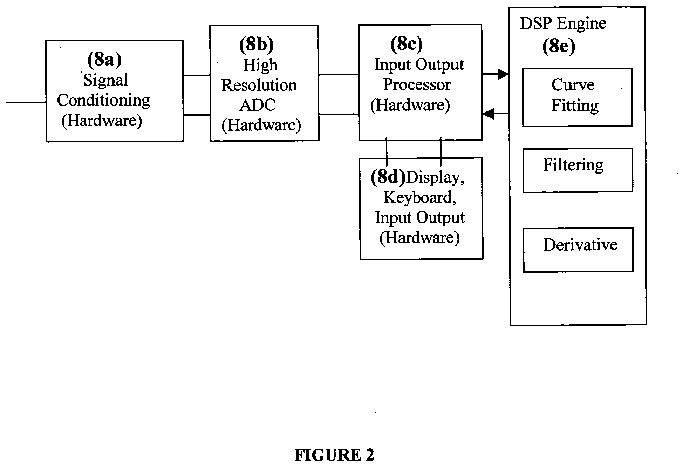

The apparatus of the present invention is illustrated in figure 1 of the accompanying drawing. Figure 2 represents the block diagram of the electronic device.

The apparatus of the present invention comprises of well or mould or refractory cup structure(2), cavity(1), thermocouple wire(4), quartz tube(3), base(6), tellurium(5), holder(7), compensating cable(9) and electronic device(8).

The refractory cup structure or mould(2) is made from resin coated sand. The sand withstands high temperature of 1050 deg C to 1400 deg C as it is refractory in nature. The diameter and height of the cup structure(2) is around 20 to 40 mm and 10 to 25 mm respectively such that the weight of the metal in the cup is about 50 to 180 gm.

The K type (CR-AL) 22 to 24 swg thermocouple wire(4) is used for measuring the temperature. Quartz tube shell(3) is fitted horizontally in the cup structure(2) such that it covers CR-AL wire(4). The quartz tube(3) avoids contact of liquid ferrous metal and thermocouple wire(4) and eliminate possibility of contamination. The quartz tube(3) is sealed with refractory agents so that there is no leakage from hole of cup(2).

Chilling agents such as Bismuth, Boron, Cerium, Lead, Magnesium and Tellurium (5) are mixed with refractory binders is pasted at the bottom of the cup as chilling agent. The quantity of chilling agents used is 0.20 to 0.50 gm. (0.2 to 0.6% by weight).

The refractory cup(2) has a suitable base(6) so as to fit it to the holder(7). This holder then carries signal to the electronic device (8) via compensating cable(9) for further analysis of percentage of Carbon, Silicon and Carbon equivalent.

An electronic device(8) capable of sensing thermal arrest points at high cooling rates is connected to the holder(7) through a compensating cable(9).
This electronic device(8) finds the Liquidus and solidus temperature as per algorithm, store, convert and display corresponding values of %CE, %Carbon and % Si on the display.

Electronic device (8) comprises of signal conditioning hardware (8a), analog to digital converter (8b), input output processor (8c), display (8d), and digital signal processor (8e).

**Method:**

The liquid ferrous metal sample is poured in a cavity (1) of cup(2). The maximum temperature as recorded by the cup(2) is stored in the electronic device (8) and further cooling is scanned. The heat liberated when austenite starts to precipitate produces an isothermal arrest on the cooling curve. During solidification in the cup, latent heat is given out. Due to the effect of natural cooling and liberation of latent heat, a thermal equilibrium is reached and a thermal arrest is obtained. This temperature is called as Liquidus temperature (TL). The arrest found, according to this invention is relatively weak due to faster cooling rate. This weak arrest is due to lower weight of sample and hence lower latent heat available to arrest temperature. The liquidus temperature being inversely proportional to the % carbon equivalent (CE), determine the % CE value empirically.

The sample gets chilled with the help of chilling agents (5) coating at the bottom of the cup (2) from inside the cavity and the sample converted into white iron. When all the liquid metal solidifies one more thermal arrest is obtained. This temperature is called Solidus temperature (TS). The time required for analysis to complete is about 50 to 80 seconds.

It is a property of any substance to have a fixed freezing point. But Cast iron, S.G Iron, malleable iron that is under consideration of present invention is exception to it. Generally iron of the consideration in present invention solidifies showing grey structure when fractured. In such case, iron with same composition solidifies at different temperature depending upon nucleation. More the nucleation, higher is the freezing point. But when it is allowed to cool fast, it solidifies giving white fracture. It is called metastable solidification. Iron with same composition solidifies at a unique temperature if it is allowed to solidify at metastable solidification temperature.

The universal Iron Carbon diagram / Iron Carbon Silicon diagram shows different solidification compositions for different values of liquidus and solidus temperature for white solidification.

The present invention makes use of metastable solidification. The metal is forced to cool fast using chilling agents. This causes metastable solidification to occur. The instrument senses solidification temperature of the iron. A table of different values of solidification temperatures verses their corresponding composition is fed in the instrument. The algorithm searches for stored liquidus and solidus temperature values and locates corresponding values of % Carbon Equivalent, % Carbon.

The value of % Si is calculated by using formula

$$\% \text{ Carbon Equivalent} = \% \text{ Carbon} + (1/3)^* \% \text{ Silicon.}$$

Smaller quantity of sample considered in the present invention cool faster than conventional sample quantity. Hence present invention uses the instrument, which can process higher cooling rates.

The faster cooling rates are measured due to the lower quantity of the sample under test. The hardware and the algorithm used in the present invention can handle cooling rates of 0 to 3 $^0$C/sec while finding liquidus and solidus temperatures.

The method using hardware and algorithm for the complete process is described in details herein.

1. When liquid metal is poured in the cup(2), the thermocouple inside the cup(2) gets heated up and generates signal in millivolts.

2. Signal conditioning(8a) of the millivolts is carried out using various components like filters and capacitors.

3. Analog signal is converted to digital signal using high resolution analog to digital converter(8b) so that input output processor(8c) can process it.

4. Store all the points of cooling process in an array using input output processor(8c).

5. A curve is generated using digital signal processor(8e) engine. Interpolation of the intermediate points is done to smoothen the curve.

6. The instantaneous cooling rate i.e 1$^{st}$ derivative at each point of the smoothened cooling curve is done by digital signal processor(8e) engine and the cooling rate values are stored in another array.

7. A filter is applied by the digital signal processor(8e) engine to fit a smooth curve for 1$^{st}$ derivative graph by interpolation.

8. 2$^{nd}$ derivative at each point of the smoothened 1$^{st}$ derivative curve is found and the 2$^{nd}$ derivative values are stored in another array.

9. A filter is applied by the digital signal processor(8e) engine to fit a smooth curve for 2$^{nd}$ derivative graph by interpolation and the values obtained are stored in another array.

10. 3$^{rd}$ derivative at each point of the smoothened 2$^{nd}$ derivative curve is found with the help of digital signal processor(8e) engine and the 3$^{rd}$ derivative values are stored in another array.

11. Maxima, minima and zero crossover points of cooling rate, 1$^{st}$ and 2$^{nd}$ derivative curves are found by using digital signal processor(8e) engine.

12. liquidus temperature and solidus temperature are detected using above mentioned points.

Liquidus and solidus point detection: When iron containing Carbon and silicon solidifies, it does so over the range of temperature instead of solidifying at a particular freezing point. When material is poured in the cup(2), the electronic device(8) senses the maximum temperature. When material is allowed to cool, initially it starts cooling at maximum cooling rate. When the temperature reaches the solidification temperature, few molecules start to solidify to precipitate austenite and thus give out latent heat of solidification. The resultant of natural cooling of material and evaluation of latent heat reduce the cooling rate of solidifying metal. Depending upon the quantity of latent heat available with the solidifying metal, the cooling rate start falling down, reach to a minimum level and start raising again. The temperature of lowest achieved cooling rate is the liquidus temperature. Since the reading are stored as time V/s temperature, the first derivative of these points is cooling rate and $2^{nd}$ derivative is rate of change of cooling rate. Therefore when the $2^{nd}$ derivative passes through zero the minima on the cooling rate curve is obtained. Corresponding temperature is the liquidus temperature.

With the same principle the solidus temperature is found. When material cool further, it reaches a temperature where the material is completely solid. It again gives out heat and the cooling rate drop again. This change in cooling rate is sensed and latched as solidus temperature.

Using algorithm, cooling rate from 0 to 3 deg. C./Sec.can be measured, handled, analyzed used by input output processor(8c) of electronic device(8) for detecting liquidus and solidus temperatures.

13. The empirical table of temperature verses corresponding % carbon equivalent and % carbon are stored in input output processor using iron carbon diagram. Input output processor(8c) find corresponding value of carbon equivalent by using liquidus temperature and display its value on the electronic device(8).

14. Input output processor(8c) is used to find %Silicon using following formula. % Carbon Equivalent= % Carbon + (1/3)* % Silicon.

15. Input output processor(8c) send values of % carbon & % silicon, liquidus and solidus temperature and display values on the display(8d) of electronic device(8).

The important processing in this hardware and algorithm essentially lies in step 5, where a filter is applied and a smooth curve fit is generated. This algorithm ensures more precise values when working with higher cooling rate. The algorithm is capable of detecting liquidus temperature and solidus temperature upto cooling rate of 3 deg./sec while finding liquidus and solidus temperatures.

Detailed descriptions of the preferred embodiment are provided herein; however, it is to be understood that the present invention may be embodied in various forms. Therefore, specific details disclosed herein are not to be interpreted as limiting, but rather as a basis for the claims and as a representative basis for teaching one skilled in the art to employ the present invention in virtually any appropriately detailed system, structure or matter.

The embodiments of the invention as described above and the method disclosed herein will suggest further modification and alterations to those skilled in the art. Such further modifications and alterations may be made without departing from the spirit and scope of the invention; which is defined by the scope of the following claims.

## Claims

1. An apparatus to determine the percentage of the Carbon Equivalent, Carbon and Silicon in a liquid ferrous metal sample; said apparatus comprising a refractory cup structure (2); a cavity (1); a thermocouple wire (4); a quartz tube shell (3); a base (6); chilling agents (5), a holder (7), a compensating cable (9), and an electronic device(8); wherein the refractory cup structure (2) is made of sand coated with resin; the diameter and height of said cup structure are proportioned to lie within the range of 20 to 40 mm and 10 to 25 mm respectively; the cavity (1) dimensioned to accommodate a weight of the liquid metal sample between about 50 to 180 gm in weight;
the thermocouple wire (4) is capable of temperature measurement of said liquid ferrous metal sample in the range of 1050 to 1400 deg C;
said quartz tube shell (3) is arranged horizontally within said cup structure (2) such that it covers said thermocouple wire (4); said quartz tube shell (3) preventing contact between said liquid ferrous metal and said thermocouple wire (4) and eliminating the possibility of contamination; said quartz tube shell (3) sealed with refractory agents to prevent leakage from hole of said cup structure (2);
a mixture of refractory binders and 0.20 to 0.50 gm chilling agents (5) arranged at the bottom of said refractory cup structure; said cup (2) possessing a suitable base (6) and being suitably dimensioned to fit the holder (7); said holder (7) being arranged to carry the measurement signal to the electronic device (8) via said compensating cable (9) for further analysis of the percentage of Carbon equivalent, Carbon and Silicon in said liquid ferrous metal sample.

2. An apparatus as claimed in claim 1 where the said electronic device (8) comprises signal conditioning hardware (8a);

an analog to digital converter(8b);
an input output processor(8c);

a display (8d); and
a digital signal processor(8e).

3. An apparatus as claimed in claim 1 where the cup structure is polygonal.

4. An apparatus as claimed in claim 1 wherein i the chilling agents are selected from. tellurium, Bismuth, Boron, Cerium, Lead, and Magnesium.

5. An apparatus as claimed in claim 1 wherein the thermocouple wire is a K type (CR-AL thermocouple wire.

6. An apparatus as claimed in claim 1 wherein the measurement of the percentage of the Carbon Equivalent, Carbon and Silicon takes from 50 to 180 seconds.

7. A method of using the apparatus as claimed in claim 1 and claim 2 for determining the percentage of the Carbon equivalent, Carbon and Silicon in a liquid ferrous metal sample comprising the steps of:

a. Pouring the liquid metal sample into said cup (2), thereby heating said thermocouple wire inside said cup (2) and generating a signal in millivolts;
b. Carrying out signal conditioning (8a) of the generated thermocouple signal in millivolts using various components like filters and capacitors;
c. Converting the analog signal to a digital signal using a high resolution analog to digital converter (8b) thereby enabling processing by the input output processor (8c).;
d. Storing all the points of the cooling process in an array using input output processor (8c);
e. Generating a smoothed curve using a digital signal processor(8e) engine; by interpolation of the intermediate points;
f. Storing in another array the cooling rate and the instantaneous cooling rate i.e 1st derivative at each point of the smoothed cooling curve;
g. Applying a filter using said digital signal processor (8e) engine to fit a smooth curve for 1st derivative graph by interpolation; wherein the first derivative represents the cooling rate;
h. Finding the 2nd derivative at each point of the smoothed 1st derivative curve and storing in another array the 2nd derivative values; wherein the 2nd derivative represents the rate of change of cooling rate
i. Applying a filter using said digital signal processor (8e) engine to fit a smooth curve for 2nd derivative graph by interpolation and storing the values obtained in another array;
j. Finding the 3rd derivative at each point of the smoothed 2nd derivative curve using said digital signal processor (8e) engine and storing the 3rd derivative values in another array;
k. Finding the maxima, minima and zero crossover points of cooling rate, 1st and 2nd derivative curves using said digital signal processor (8e) engine;
l. Detecting the liquidus temperature and solidus temperature using above mentioned points; wherein

(i) the liquidus temperature is the temperature of the lowest achieved cooling rate identified by the point where the 2nd derivative obtained in (h) passes through zero so the minima on the cooling rate curve is obtained; and
(ii) the solidus temperature is the temperature where the material is completely solid identified as the temperature at which the material gives out heat and a further drop in cooling rate sensed;
(iii) the liquidus temperature and the solidus temperature can be detected by the input output processor (8c) of said electronic device (8) using an algorithm to measure, handle, and analyse a cooling rate from 0 to 3 deg. C./Sec; as

(A) when iron containing Carbon and silicon solidifies, it does so over the range of temperature instead of solidifying at a particular freezing point;
(B) when the sample is poured in said cup (2), said electronic device (8) senses the maximum temperature;
(C) when the sample is allowed to cool, initially it starts cooling at maximum cooling rate;
(D) when the temperature reaches the solidification temperature, a few molecules start to solidify to precipitate austenite and thus give out latent heat of solidification;
(E) the resultant natural cooling of the sample and evaluation of latent heat reduce the cooling rate of the solidifying metal sample; depending upon the quantity of latent heat available with the solidifying metal sample, the cooling rate starts to reduce, reaching a minimum level and then starts increasing again;

(F) since the measurements obtained are stored as time versus temperature, the first derivative of these points is cooling rate and 2nd derivative is rate of change of cooling rate;

(G) when the 2nd derivative passes through zero, the minima on the cooling rate curve is obtained and the corresponding temperature is the liquidus temperature; and

(H) when the sample cools further, it reaches a temperature where the sample is completely solid; and it again gives out heat and the cooling rate drops again; this change in cooling rate is sensed and the solidus temperature identified;

m. Storing in said input output processor(8c) the empirical table of temperature versus corresponding % carbon equivalent and % carbon using iron carbon diagram; using said input output processor (8c) to find the corresponding value of carbon equivalent by using liquidus temperature and display its value on the said electronic device (8);

n. Using the input output processor (8c) to find the %Silicon using the following formula:

$$\text{\% Carbon Equivalent} = \text{\% Carbon} + (1/3)^* \text{ \% Silicon;}$$

o. Sending values of % carbon & % silicon, liquidus and solidus temperature from said input output processor (8c) and displaying values on the display(8d) of said electronic device(8).

## Patentansprüche

1. Vorrichtung zum Bestimmen des prozentualen Anteils des Kohlenstoffäquivalents, des Kohlenstoffs und des Siliziums in einer flüssigen Eisenmetallprobe; wobei die Vorrichtung umfasst: eine feuerfeste Becherstruktur (2); einen Hohlraum (1); einen Thermoelementdraht (4); einen Quarzrohrmantel (3); eine Basis (6); Kühlmittel (5), einen Halter (7), ein Kompensationskabel (9) und eine elektronische Vorrichtung (8); wobei

die feuerfeste Becherstruktur (2) aus mit Harz beschichtetem Sand hergestellt ist; der Durchmesser und die Höhe der Becherstruktur so bemessen sind, dass sie im Bereich von 20 bis 40 mm bzw. 10 bis 25 mm liegen; der Hohlraum (1) zur Aufnahme eines Gewichts der flüssigen Metallprobe zwischen etwa 50 und 180 g dimensioniert ist;

der Thermoelementdraht (4) in der Lage ist, die Temperatur der flüssigen Eisenmetallprobe im Bereich von 1050 bis 1400 °C zu messen;

der Quarzrohrmantel (3) horizontal innerhalb der Becherstruktur (2) angeordnet ist, derart, dass er den Thermoelementdraht (4) bedeckt; der Quarzrohrmantel (3) einen Kontakt zwischen dem flüssigen Eisenmetall und dem Thermoelementdraht (4) verhindert und die Möglichkeit einer Kontamination ausschließt; der Quarzrohrmantel (3) mit feuerfesten Mitteln abgedichtet ist, um eine Leckage durch ein Loch der Becherstruktur (2) zu verhindern;

eine Mischung aus feuerfesten Bindemitteln und 0,20 bis 0,50 g Kühlmitteln (5), die am Boden der feuerfesten Becherstruktur angeordnet ist; wobei der Becher (2) eine geeignete Basis (6) aufweist und geeignet dimensioniert ist, um auf den Halter (7) zu passen; wobei der Halter (7) dazu eingerichtet ist, das Messsignal über das Kompensationskabel (9) zur weiteren Analyse des Prozentsatzes von Kohlenstoffäquivalent, Kohlenstoff und Silizium in der flüssigen Eisenmetallprobe an die elektronische Vorrichtung (8) zu leiten.

2. Vorrichtung nach Anspruch 1, wobei die elektronische Vorrichtung (8) umfasst:

Hardware zur Signalaufbereitung (8a);
einen Analog-Digital-Wandler (8b);
einen Eingabe-Ausgabe-Prozessor (8c);
eine Anzeige (8d); und
einen digitalen Signalprozessor (8e).

3. Vorrichtung nach Anspruch 1, wobei die Becherstruktur polygonal ist.

4. Vorrichtung nach Anspruch 1, wobei die Kühlmittel ausgewählt sind aus Tellur, Bismut, Bor, Cer, Blei und Magnesium.

5. Vorrichtung nach Anspruch 1, wobei der Thermoelementdraht ein Thermoelementdraht vom Typ K (CR-AL) ist.

6. Vorrichtung nach Anspruch 1, wobei die Messung des Prozentsatzes des Kohlenstoffäquivalents, des Kohlenstoffs und des Siliziums zwischen 50 und 180 Sekunden dauert.

7. Verfahren zum Verwenden der Vorrichtung nach Anspruch 1 und Anspruch 2 zum Bestimmen des Prozentsatzes des Kohlenstoffäquivalents, des Kohlenstoffs und des Siliziums in einer flüssigen Eisenmetallprobe, umfassend die folgenden Schritte:

a. Gießen der flüssigen Metallprobe in den Becher (2), wodurch der Thermoelementdraht im Inneren des Bechers (2) erhitzt wird und ein Signal in Millivolt erzeugt wird;

b. Durchführen von Signalaufbereitung (8a) des erzeugten Thermoelementsignals in Millivolt unter Verwendung verschiedener Komponenten wie Filtern und Kondensatoren;

c. Wandeln des analogen Signals in ein digitales Signal unter Verwendung eines hochauflösenden Analog-DigitalWandlers (8b), wodurch die Verarbeitung durch den Eingabe-Ausgabe-Prozessor (8c) ermöglicht wird;

d. Speichern aller Punkte des Kühlprozesses in einem Array unter Verwendung des Eingabe-Ausgabe-Prozessors (8c) ;

e. Erzeugen einer geglätteten Kurve unter Verwendung einer Engine des digitalen Signalprozessors (8e); durch Interpolation der Zwischenpunkte;

f. Speichern, in einem weiteren Array, der Abkühlungsrate und der momentanen Abkühlungsrate, d. h. der 1. Ableitung an jedem Punkt der geglätteten Abkühlungskurve;

g. Anwenden eines Filters unter Verwendung der Engine des digitalen Signalprozessors (8e) zum Anpassen einer glatten Kurve für den Graphen der 1. Ableitung durch Interpolation; wobei die erste Ableitung die Abkühlungsrate repräsentiert;

h. Ermitteln der 2. Ableitung an jedem Punkt der geglätteten Kurve der 1. Ableitung und Speichern, in einem weiteren Array, der Werte der 2. Ableitung; wobei die 2. Ableitung die Änderungsrate der Abkühlungsrate repräsentiert.

i. Anwenden eines Filters unter Verwendung der Engine des digitalen Signalprozessors (8e) zum Anpassen einer glatten Kurve für den Graphen der 2. Ableitung durch Interpolation und Speichern der erhaltenen Werte in einem weiteren Array;

j. Ermitteln der 3. Ableitung an jedem Punkt der geglätteten Kurve der 2. Ableitung unter Verwendung der Engine des digitalen Signalprozessors (8e) und Speichern der Werte der 3. Ableitung in einem weiteren Array;

k. Ermitteln der Maxima, Minima und Nulldurchgangspunkte der Kurven von Abkühlungsrate und 1. und 2. Ableitung unter Verwendung der Engine des digitalen Signalprozessors (8e);

l. Erfassen der Liquidustemperatur und der Solidustemperatur unter Verwendung der oben genannten Punkte; wobei

(i) die Liquidustemperatur die Temperatur der niedrigsten erreichten Abkühlungsrate ist, die durch den Punkt identifiziert wird, an dem die in (h) erhaltene 2. Ableitung durch Null geht, wodurch man das Minimum auf der Abkühlungsratenkurve erhält; und

(ii) die Solidustemperatur die Temperatur ist, bei der das Material vollständig fest ist, identifiziert als die Temperatur, bei der das Material Wärme abgibt und ein weiterer Abfall der Abkühlungsrate gemessen wird;

(iii) die Liquidustemperatur und die Solidustemperatur durch den Eingabe-Ausgabe-Prozessor (8c) der elektronischen Vorrichtung (8) unter Verwendung eines Algorithmus zum Messen, Verarbeiten und Analysieren einer Abkühlungsrate von 0 bis 3 °C/Sekunde über folgende Sachverhalte erfasst werden können:

(A) Wenn kohlenstoff- und siliziumhaltiges Eisen erstarrt, geschieht dies über einen Temperaturbereich, anstatt bei einem spezifischen Erstarrungspunkt zu erstarren;

(B) wenn die Probe in den Becher (2) gegossen wird, erfasst die elektronische Vorrichtung (8) die maximale Temperatur;

(C) wenn man die Probe abkühlen lässt, beginnt sie zunächst mit maximaler Abkühlungsrate abzukühlen;

(D) wenn die Temperatur die Erstarrungstemperatur erreicht, beginnen einige wenige Moleküle unter Ausfällen von Austenit zu erstarren und somit latente Erstarrungswärme abzugeben;

(E) die resultierende natürliche Abkühlung der Probe und die Auswertung von latenter Wärme reduzieren die Abkühlungsrate der erstarrenden Metallprobe; je nach der Menge an latenter Wärme, die in der erstarrenden Metallprobe verfügbar ist, beginnt die Abkühlungsrate zu sinken, erreicht einen minimalen Wert und beginnt dann wieder anzusteigen;

(F) da die erhaltenen Messungen als Zeit gegen Temperatur gespeichert werden, ist die erste Ableitung dieser Punkte die Abkühlungsrate und die 2. Ableitung die Änderungsrate der Abkühlungsrate;

(G) wenn die 2. Ableitung durch Null geht, erhält man das Minimum auf der Abkühlungsratenkurve, und die entsprechende Temperatur ist die Liquidustemperatur; und

(H) wenn die Probe weiter abkühlt, erreicht sie eine Temperatur, bei der die Probe vollständig fest ist; und sie gibt erneut Wärme ab und die Abkühlungsrate sinkt erneut; diese Änderung der Abkühlungsrate wird erfasst und die Solidustemperatur wird identifiziert;

m. Speichern, im Eingabe-Ausgabe-Prozessor (8c), der empirischen Tabelle von Temperatur gegen den entsprechenden prozentualen Anteil von Kohlenstoffäquivalent und den entsprechenden prozentualen Anteil von Kohlenstoff unter Verwendung eines Eisen-Kohlenstoff-Diagramms; Verwenden des Eingabe-Ausgabe-Prozessors (8c) zum Ermitteln des entsprechenden Werts von Kohlenstoffäquivalent unter Verwendung der Liquidustemperatur und Anzeigen seines Werts auf der elektronischen Vorrichtung (8);

n. Verwenden des Eingabe-Ausgabe-Prozessors (8c) zum Ermitteln des prozentualen Anteils von Silizium unter Verwendung der folgenden Formel:

```
Prozentualer   Anteil   von   Kohlenstoffäquivalent   =
prozentualer Anteil von Kohlenstoff + (1/3)* prozentualer
Anteil von Silizium;
```

o. Senden von Werten des prozentualen Anteils von Kohlenstoff und des prozentualen Anteils von Silizium, der Liquidus- und der Solidustemperatur vom Eingabe-Ausgabe-Prozessor (8c) und Anzeigen von Werten auf dem Display (8d) der elektronischen Vorrichtung (8).

**Revendications**

1. Appareil pour déterminer le pourcentage de l'équivalent carbone, du carbone et du silicium dans un échantillon de métal ferreux liquide ; ledit appareil comprenant une structure de coupelle réfractaire (2) ; une cavité (1) ; un fil de thermocouple (4) ; une enveloppe tubulaire en quartz (3) ; une base (6) ; des agents de refroidissement (5), un support (7), un câble de compensation (9) et un dispositif électronique (8) ; dans lequel

   la structure de coupelle réfractaire (2) est constituée de sable revêtu de résine ; le diamètre et la hauteur de ladite structure de coupelle sont proportionnés pour se situer dans la plage de 20 à 40 mm et de 10 à 25 mm respectivement ;

   la cavité (1) est dimensionnée pour contenir un poids de l'échantillon de métal liquide compris entre environ 50 et 180 g en poids ;

   le fil de thermocouple (4) est apte à la mesure de température dudit échantillon de métal ferreux liquide dans la plage de 1050 à 1400 °C ;

   ladite enveloppe tubulaire en quartz (3) est disposée horizontalement au sein de ladite structure de coupelle (2) de façon telle qu'elle recouvre ledit fil de thermocouple (4) ; ladite enveloppe tubulaire en quartz (3) empêchant le contact entre ledit métal ferreux liquide et ledit fil de thermocouple (4) et éliminant la possibilité de contamination ; ladite enveloppe tubulaire en quartz (3) étant hermétiquement scellée avec des agents réfractaires pour empêcher une fuite à partir de l'ouverture de ladite structure de coupelle (2) ;

   un mélange de liants réfractaires et de 0,20 à 0,50 g d'agents de refroidissement (5) est disposé au fond de ladite structure de coupelle réfractaire ; ladite coupelle (2) possédant une base (6) appropriée et étant dimensionnée de manière appropriée pour s'ajuster dans le support (7) ; ledit support (7) étant conçu pour transmettre le signal de mesure au dispositif électronique (8) par l'intermédiaire dudit câble de compensation (9) pour analyse complémentaire du pourcentage d'équivalent carbone, de carbone et de silicium dans ledit échantillon de métal ferreux liquide.

2. Appareil tel que revendiqué dans la revendication 1 où ledit dispositif électronique (8) comprend

   du matériel de conditionnement de signal (8a) ;

   un convertisseur analogique-numérique (8b) ;

   un processeur d'entrée-sortie (8c) ;

   un afficheur (8d) ; et

   un processeur de signal numérique (8e).

3. Appareil tel que revendiqué dans la revendication 1 où la structure de coupelle est polygonale.

4. Appareil tel que revendiqué dans la revendication 1 dans lequel les agents de refroidissement sont choisis parmi le tellure, le bismuth, le bore, le cérium, le plomb et le magnésium.

5. Appareil tel que revendiqué dans la revendication 1 dans lequel le fil de thermocouple est un fil de thermocouple de type K (CR-AL).

6. Appareil tel que revendiqué dans la revendication 1 dans lequel la mesure du pourcentage de l'équivalent carbone, du carbone et du silicium prend de 50 à 180 secondes.

7. Procédé d'utilisation de l'appareil tel que revendiqué dans la revendication 1 et la revendication 2 pour la détermination du pourcentage de l'équivalent carbone, du carbone et du silicium dans un échantillon de métal ferreux liquide comprenant les étapes consistant à :

> a. verser l'échantillon de métal liquide dans ladite coupelle (2), ce qui chauffe de cette manière ledit fil de thermocouple à l'intérieur de ladite coupelle (2) et produit un signal en millivolts ;
> b. mettre en œuvre un conditionnement de signal (8a) du signal de thermocouple produit en millivolts à l'aide de divers composants comme des filtres et des condensateurs ;
> c. convertir le signal analogique en un signal numérique à l'aide d'un convertisseur analogique-numérique à haute résolution (8b) ce qui permet de cette manière le traitement par le processeur d'entrée-sortie (8c) ;
> d. stocker tous les points du processus de refroidissement dans un tableau à l'aide du processeur d'entrée-sortie (8c) ;
> e. produire une courbe lissée à l'aide d'un moteur de processeur de signal numérique (8e) ; par interpolation des points intermédiaires ;
> f. stocker dans un autre tableau la vitesse de refroidissement et la vitesse de refroidissement instantanée c'est-à-dire la dérivée première en chaque point de la courbe de refroidissement lissée ;
> g. appliquer un filtre à l'aide dudit moteur de processeur de signal numérique (8e) pour ajuster une courbe lisse pour le graphique de la dérivée première par interpolation ; la dérivée première représentant la vitesse de refroidissement ;
> h. trouver la dérivée seconde en chaque point de la courbe de dérivée première lissée et stocker dans un autre tableau les valeurs de dérivée seconde ; la dérivée seconde représentant la vitesse de variation de vitesse de refroidissement
> i. appliquer un filtre à l'aide dudit moteur de processeur de signal numérique (8e) pour ajuster une courbe lisse pour le graphique de la dérivée seconde par interpolation et stocker les valeurs obtenues dans un autre tableau ;
> j. trouver la dérivée troisième en chaque point de la courbe de dérivée seconde lissée à l'aide dudit moteur de processeur de signal numérique (8e) et stocker les valeurs de dérivée troisième dans un autre tableau ;
> k. trouver les points de maximum, de minimum et de passage par zéro des courbes de vitesse de refroidissement, de dérivée première et de dérivée seconde à l'aide dudit moteur de processeur de signal numérique (8e) ;
> l. repérer la température de liquidus et la température de solidus à l'aide des points mentionnés ci-dessus ;

>> (i) la température de liquidus étant la température de la plus faible vitesse de refroidissement atteinte identifiée par le point où la dérivée seconde obtenue en (h) passe par zéro de sorte que le minimum sur la courbe de vitesse de refroidissement est obtenu ; et
>> (ii) la température de solidus étant la température où le matériau est complètement solide identifiée comme la température à laquelle le matériau dégage de la chaleur et une chute supplémentaire de vitesse de refroidissement est détectée ;
>> (iii) la température de liquidus et la température de solidus pouvant être repérées par le processeur d'entrée-sortie (8c) dudit dispositif électronique (8) à l'aide d'un algorithme pour mesurer, traiter et analyser une vitesse de refroidissement de 0 à 3 °C/s ; étant donné que

>>> (A) lorsque du fer contenant du carbone et du silicium se solidifie, il le fait sur la plage de température plutôt que de se solidifier à un point de congélation particulier ;
>>> (B) lorsque l'échantillon est versé dans ladite coupelle (2), ledit dispositif électronique (8) détecte la température maximale ;
>>> (C) lorsque l'échantillon est amené à refroidir, initialement il commence à refroidir à la vitesse de refroidissement maximale ;
>>> (D) lorsque la température atteint la température de solidification, quelques molécules commencent à se solidifier pour faire précipiter de l'austénite et donnent ainsi de la chaleur latente de solidification ;
>>> (E) le refroidissement naturel résultant de l'échantillon et l'évaluation de chaleur latente réduisent la vitesse de refroidissement de l'échantillon de métal en train de se solidifier ; selon la quantité de chaleur latente disponible avec l'échantillon de métal en train de se solidifier, la vitesse de refroidissement commence à baisser, atteignant un niveau minimal puis recommence à augmenter ;

(F) puisque les mesures obtenues sont stockées sous forme de température en fonction du temps, la dérivée première de ces points est la vitesse de refroidissement et la dérivée seconde est la vitesse de variation de vitesse de refroidissement ;

(G) lorsque la dérivée seconde passe par zéro, le minimum sur la courbe de vitesse de refroidissement est obtenu et la température correspondante est la température de liquidus ; et

(H) lorsque l'échantillon refroidit encore, il atteint une température où l'échantillon est complètement solide ; et il dégage à nouveau de la chaleur et la vitesse de refroidissement chute à nouveau ; cette variation de vitesse de refroidissement est détectée et la température de solidus est identifiée ;

m. stocker dans ledit processeur d'entrée-sortie (8c) la table empirique du % d'équivalent carbone et du % de carbone correspondants en fonction de la température à l'aide du diagramme fer-carbone ; utiliser ledit processeur d'entrée-sortie (8c) pour trouver la valeur correspondante d'équivalent carbone à l'aide de la température de liquidus et afficher sa valeur sur ledit dispositif électronique (8) ;

n. utiliser le processeur d'entrée-sortie (8c) pour trouver le % de silicium à l'aide de la formule suivante :

```
% d'équivalent carbone = % de carbone + (1/3)*% de
silicium ;
```

o. envoyer les valeurs de % de carbone et de % de silicium, de température de liquidus et de solidus provenant dudit processeur d'entrée-sortie (8c) et afficher les valeurs sur l'afficheur (8d) dudit dispositif électronique (8).

**FIGURE 1**

**FIGURE 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5503475 A
- US 5720553 A
- US 4358948 A
- US 3404570 A
- US 3546921 A
- US 4059996 A
- US 4515485 A
- US 4274284 A
- US 6739750 B